# EUROPÄISCHE PATENTSCHRIFT

(11) **EP 0 560 444 B1**
(45) Veröffentlichungstag und Bekanntmachung des Hinweises auf die Patenterteilung: **11.06.1997**
(21) Anmeldenummer: 93200649.7
(22) Anmeldetag: 08.03.1993
(51) Int. Cl.: A61B 17/225, A61B 5/0468, A61B 5/0456

(54) **Anordnung zur Stosswellenbehandlung**
Shock wave treatment device
Dispositif de traitement par ondes de choc

(30) Priorität: 10.03.1992 DE 4207489
(43) Veröffentlichungstag der Anmeldung: 15.09.1993
(73) Patentinhaber: Philips Patentverwaltung GmbH, 22335 Hamburg (DE); Philips Electronics N.V., 5621 BA Eindhoven (NL)
(72) Erfinder: Schwarze, Werner, c/o Philips Patentverwaltung, W-2000 Hamburg 1 (DE); Voss, Joachim, c/o Philips Patentverwaltung GmbH, W-2000 Hamburg 1 (DE)
(74) Vertreter: Hartmann, Heinrich, Dipl.-Ing.

(56) Entgegenhaltungen:
- EP-A- 0 081 051
- EP-A- 0 429 109
- DE-A- 2 304 173

## Beschreibung

Die Erfindung betrifft eine Anordnung zur Stoßwellenbehandlung mit einem Stoßwellenerzeuger und Mitteln zur Erzeugung eines Herzsignals, das der Herzaktion des Patienten entspricht. Eine derartige Anordnung ist aus der DE-PS 31 46 628 bekannt. Die Herzsignale werden dabei von einer EKG-Sonde geliefert. Sie werden benötigt, um eine bestimmte zeitliche Korrelation zwischen den EKG-Signalen und den Stoßwellenimpulsen zu erreichen, wodurch Extrasystolen, d.h. außerhalb des regulären Herzrhythmus auftretende Herzschläge, vermieden werden sollen. Die Dauer einer Stoßwellenbehandlung, in deren Verlauf eine Vielzahl von Stoßwellen appliziert wird, hängt somit von dem Herzrhythmus des Patienten ab.

Aufgabe der vorliegenden Erfindung ist es, eine Anordnung der eingangs genannten Art so auszugestalten, daß Kreislautkomplikationen bei einer Stoßwellenbehandlung ebenfalls verhindert werden. Diese Aufgabe wird erfindungsgemäß gelöst durch
- einen Analysator zum Ermitteln von Extrasystolen im Herzsignal,
- eine dem Analysator nachgeschaltete Schaltung zur Bestimmung der Häufigkeit der Extrasystolen und
- von dieser Schaltung gesteuerte Mittel zum Steuern des Stoßwellenerzeugers in Abhängigkeit der Häufigkeit der Extrasystolen.

Die Frequenz der Stoßwellenerzeugung ist bei der Erfindung unabhängig vom Herzrhythmus bzw. von der Pulsfrequenz des Patienten. Sie kann daher höher sein als es bei der bekannten Anordnung mit EKG-synchroner Stoßwellenerzeugung möglich ist, so daß sich kürzere Behandlungszeiten ergeben.

Bei einer erfindungsgemäßen Anordnung können Extrasystolen entstehen, wenn ein Stoßwellenimpuls zeitlich in die erregbare Phase der Herzaktion fällt. Der Analysator erfaßt jedoch diese Extrasystolen und liefert entsprechende Signale an eine Schaltung zur Bestimmung der Häufigkeit der Extrasystolen. Durch diese Schaltung wird der Stoßwellenerzeuger in Abhängigkeit von der Häufigkeit der Extrasystolen derart gesteuert, daß bedrohliche Zustände wirksam unterbunden werden.

Dies kann auf verschiedene Weise erfolgen. Eine besonders einfache Weiterbildung der Erfindung sieht vor, daß die Mittel zum Steuern des Stoßwellenerzeugers den Stoßwellenerzeuger wenigstens vorübergehend abschalten.

Eine weitere Vereinfachung ergibt sich gemäß einer anderen Weiterbildung dadurch, daß die Schaltung zur Bestimmung der Häufigkeit der Extrasystolen einen Zähler enthält. Der Zähler zählt also die Extrasystolen und bewirkt bei Erreichen eines bestimmten Zählerstandes ein - zumindest vorübergehendes - Abschalten des Stoßwellenerzeugers. In weiterer Ausgestaltung ist vorgesehen, daß ein Zeitgeber zum Rücksetzen des Zählers nach einem definierten Zeitraum vorgesehen ist, der durch ein vom Analysator geliefertes Signal aktivierbar ist, das einer Extrasystole entspricht. In diesem Fall wird der Stoßwellenerzeuger nur beeinflußt, wenn innerhalb eines durch den Zeitgeber vorgegebenen - vorzugsweise einstellbaren - Zeitintervalls eine bestimmte Zahl von Extrasystolen auftritt. Hierbei erfolgt also eine Beeinflussung des Zeitgebers nur dann, wenn sich eine gewisse zeitliche Dichte der Extrasystolen ergibt; in größeren zeitlichen Abständen auftretende Extrasystolen sind weniger schädlich und können daher toleriert werden.

Die Erfindung wird nachstehend anhand der Zeichnung näher erläutert. Es zeigen
- Fig. 1: ein Blockschaltbild einer erfindungsgemäßen Anordnung und
- Fig. 2: EKG-Signale mit und ohne Extrasystolen.

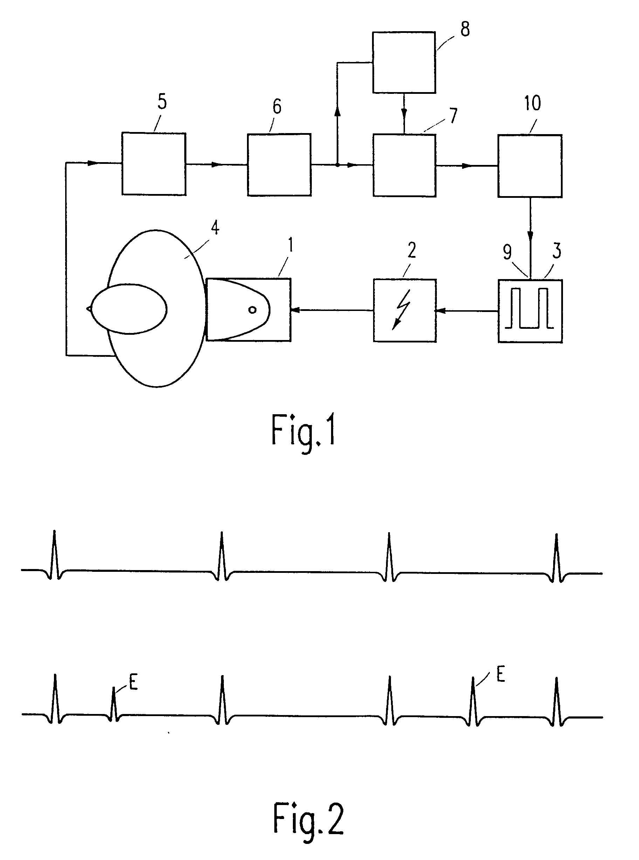

Bei der in Fig. 1 schematisch dargestellten Anordnung umfaßt der Stoßwellenerzeuger die Einheiten 1, 2 und 3. Die Einheit 1 erzeugt die eigentliche Stoßwelle. Sie kann ein Rotationsellipsoid aufweisen, in dessen einem Brennpunkt sich eine Funkenstrecke befindet bei deren Zündung impulsartig eine Stoßwelle entsteht. Die dabei erzeugte Stoßwelle wird auf den zweiten Brennpunkt des Rotationsellipsoids fokussiert, in dem sich bei einer Stoßwellenbehandlung ein zu zertrümmerndes Körperkonkrement eines Patienten 4, beispielsweise ein Nierenstein, befindet. Es kann jedoch auch ein Stoßwellenerzeuger vorgesehen sein, der die Stoßwellen auf andere Weise erzeugt, beispielsweise mittels einer elektromagnetisch angetriebenen Membran, deren Stoßwellen mittels Reflektoren und/oder akustischer Linsen auf einen Punkt fokussiert werden.

Die Energie für die Einheit 1 wird von einem Hochspannungserzeuger 2 geliefert, der von einem Impulsgeber 3 mit vorzugsweise einstellbarer Impulsfrequenz beaufschlagt wird und bei jedem Impuls Energie für die Zündung der Funkenstrecke in der Einheit 1 liefert.

Der Patient 4, der der Stoßwellenbehandlung unterzogen wird, ist an eine Schaltung 5 angeschlossen, die der Herzaktion des Patienten entsprechende Herzsignale liefert. Dabei kann es sich ebenso wie bei der eingangs erwähnten bekannten Anordnung um einen EKG-Aufnehmer handeln. Da es auf den genauen zeitlichen Verlauf der Herzsignale nicht ankommt, können aber auch andere Schaltungen verwendet werden, die solche Signale liefern können, beispielsweise (photoelektrische) Pulsaufnehmer oder Schaltungen, die die Herztöne in elektrische Signale umsetzen.

Die Herzsignale werden einem Analysator 6 zugeführt, der Extrasystolen detektieren kann und bei jeder Extrasystole an seinem Ausgang einen Impuls erzeugt.

Fig. 2 zeigt den zeitlichen Verlauf der dem Analysator 6 zugeführten EKG-Signale, wobei in Fig. 1 ein EKG-Signal ohne Extrasystolen und in Fig. 2 ein EKG-Signal mit Extrasystolen R dargestellt sind. Die Spitzen des Signals repräsentieren dabei jeweils den sogenannten QRS-Komplex. Man erkennt, daß bei dem mit Extrasystolen E behafteten EKG-Signal in der zweiten Zeile der zeitliche Abstand der Spitzen im Vergleich zu einer von Extrasystolen freien Herzaktion deutlich abnimmt. Durch Messung des zeitlichen Abstandes aufeinanderfolgender Spitzen lassen sich daher Extrasystolen relativ leicht feststellen. EKG-Geräte, die Extrasystolen detektieren können, sind darüberhinaus bereits am Markt erhältlich, beispielsweise das Gerät des Typs SMV 104 D der Firma Hellige mit dem entsprechenden Modul.

Die vom Analysator gelieferten, jeweils eine Extrasystole E repräsentierenden Impulse werden einerseits dem Eingang eines rücksetzbaren Zählers 7 und andererseits dem Eingang eines Zeitgliedes 8 - z.B. einer monostabilen Kippschaltung zugeführt, die nach einem vorzugsweise einstellbaren Zeitintervall an ihrem mit dem Rücksetzeingang des Zählers verbundenen Ausgang einen Impuls abgibt, der den Zähler zurücksetzt. Ist innerhalb des durch das Zeitglied 8 vorgegebenen Zeitintervalls ein bestimmter zweckmäßigerweise voreinstellbarer Zählerstand nicht erreicht, dann beginnt die Zählung der Extrasystolen von neuem. Andernfalls liefert der Zähler ein Signal an eine Steuereinrichtung 10, deren Ausgang einen Steuereingang 9 des Impulsgebers 3 derart steuert, daß bei einem Signal vom Zähler 7 der Impulsgeber 3 für eine bestimmte Zeit blockiert wird.

Das vom Zeitgeber vorgegebene Zeitintervall 8 und der voreinstellbare Zählerstand, bei dessen Erreichen der Zähler 7 ein entsprechendes Signal an die Steuereinheit 10 liefert, müssen so gewählt sein, daß der Patient innerhalb des Zeitintervalls die vorgegebene Zähl von Extrasystolen komplikationslos ertragen kann. Die Dauer, für die die Steuereinrichtung 10 den Impulsgeber 3 und damit die Stoßwellenerzeugung blockiert, muß so gewählt sein, daß sich der Kreislauf des Patienten innerhalb dieser Behandlungspause regenerieren kann. Diese an den Einheiten 7, 8 und 10 einstellbaren Werte werden vom Arzt entsprechend der Konstitution des Patienten so vorgegeben, daß bei der Behandlung keine Komplikationen auftreten können.

Es kann zweckmäßig sein, nach einer ersten Pause die oben erwähnten Werte so vorzugeben, daß bereits bei einer geringeren Häufigkeit der Extrasystolen der Impulsgeber 3 blockiert wird oder daß die zweite Behandlungspause größer wird als die erste. In diesem Fall müssen die Voreinstellwerte während einer Behandlung automatisch variiert werden. Dies könnte grundsätzlich mit einer entsprechend gestalteten Schaltung realisiert werden. Zweckmäßiger ist es jedoch, die Funktion der Einheiten 7, 8 und 10 mittels eines entsprechend programmierten Mikrocomputers zu realisieren, der in einem Lithotripsiegerät ohnehin erforderlich ist und der gegebenenfalls auch noch die Funktion der Einheit 3 und/oder 6 übernehmen könnte.

Es ist auch möglich, bei mit einer bestimmten Häufigkeit auftretenden Extrasystolen die Stoßwellenenergie zu verringern, anstatt die Behandlung zu unterbrechen oder ganz abzubrechen; die Steuereinheit 10 müßte dann auf den Hochspannungserzeuger 2 einwirken. Es würde dann unter Einbeziehung des Patienten ein Regelkreis entstehen, der so eingestellt werden könnte, daß eine bestimmte Häufigkeit der Extrasystolen nicht überschritten wird.

Wenn dadurch die Häufigkeit der Extrasystolen einen kritischen Wert noch immer nicht unterschreitet, könnte der Impulserzeuger vorübergehend oder ganz abgeschaltet werden.

## Patentansprüche

1. Anordnung zur Stoßwellenbehandlung mit einem Stoßwellenerzeuger (1,2,3) und Mitteln (5) zur Erzeugung eines Herzsignals, das der Herzaktion des Patienten (4) entspricht,
gekennzeichnet durch
- einen Analysator (6) zum Ermitteln von Extrasystolen im Herzsignal,
- eine dem Analysator nachgeschaltete Schaltung (7,8) zur Bestimmung der Häufigkeit der Extrasystolen und
- von dieser Schaltung gesteuerte Mittel (9,10) zum Steuern des Stoßwellenerzeugers in Abhängigkeit der Häufigkeit der Extrasystolen.

2. Anordnung nach Anspruch 1,
dadurch gekennzeichnet daß die Schaltung zur Bestimmung der Häufigkeit der Extrasystolen einen Zähler (7) enthält.

3. Anordnung nach Anspruch 2,
dadurch gekennzeichnet, daß ein Zeitgeber (8) zum Rücksetzen des Zählers (7) nach einem definierten Zeitraum vorgesehen ist, der durch ein vom Analysator (6) geliefertes Signal aktivierbar ist, das einer Extrasystole (E) entspricht.

4. Anordnung nach einem der vorhergehenden Ansprüche,
dadurch gekennzeichnet, daß die Mittel (9,10) zum Steuern des Stoßwellenerzeugers den Stoßwellenerzeuger wenigstens vorübergehend abschalten.

5. Anordnung nach einem der vorhergehenden Ansprüche,
dadurch gekennzeichnet, daß die Mittel (9,10) zum Steuern des Stoßwellenerzeugers eine Verringerung der Energie der vom Stoßwellenerzeuger (1,2,3) erzeugten Stoßwellen bewirken.

6. Anordnung nach einem der vorherigen Ansprüche,
dadurch gekennzeichnet, daß der Stoßwellenerzeuger einen die zeitliche Folge der Stoßwellen vorgebenden Impulserzeuger (3) mit einstellbarer Pulsfrequenz enthält.

## Claims

1. A device for shockwave treatment, comprising a shockwave generator (1, 2, 3) and means (5) for generating a cardiac signal corresponding to the cardiac action of the patient (4), characterized in that it comprises
- an analyser (6) for determining extrasystoles in the cardiac signal,
- a circuit (7, 8) for determining the frequency of occurrence of the extrasystoles which is connected subsequent to the analyser, and
- means (9, 10) for controlling the shockwave generator in dependence on the frequency of occurence of the extrasystoles, which means are controlled by said circuit.

2. A device as claimed in Claim 1, characterized in that the circuit for determining the frequency of occurence of the extrasystoles comprises a counter (7).

3. A device as claimed in Claim 2, characterized in that there is provided a timer (8) for resetting the counter (7) after a defined period of time, which timer can be activated by a signal which is supplied by the analyser (6) and corresponds to an extrasystole (E).

4. A device as claimed in any one of the preceding Claims, characterized in that the means (9, 10) for controlling the shockwave generator deactivate the shockwave generator at least temporarily.

5. A device as claimed in any one of the preceding Claims, characterized in that the means (9, 10) for controlling the shockwave generator induce a reduction of the energy of the shockwaves generated by the shockwave generator (1, 2, 3).

6. A device as claimed in any one of the preceding Claims, characterized in that the shockwave generator comprises a pulse generator (3) of adjustable pulse frequency which predetermines the succession in time of the shockwaves.

## Revendications

1. Dispositif de traitement par ondes de choc avec un générateur d'ondes de choc (1, 2, 3) et des moyens (5) pour la production d'un signal cardiaque qui correspond au rythme cardiaque du patient (4), caractérisé par
- un analyseur (6) pour déterminer des extrasystoles dans le signal cardiaque,
- un circuit (7, 8) monté en aval de l'analyseur en vue de déterminer la fréquence des extrasystoles, et
- des moyens (9, 10) commandés par ce circuit pour commander le générateur d'ondes de choc en fonction de la fréquence des extrasystoles.

2. Dispositif selon la revendication 1, caractérisé en ce que le circuit pour la détermination de la fréquence des extrasystoles contient un compteur (7).

3. Dispositif selon la revendication 2, caractérisé en ce qu'un générateur de rythme (8) est prévu pour réinitialiser le compteur (7) après un intervalle de temps défini, lequel peut être activé par un signal produit par un analyseur (6) qui correspond à une extrasystole (E).

4. Dispositif selon l'une des revendications précédentes, caractérisé en ce que les moyens (9, 10) pour commander le générateur d'ondes de choc mettent hors tension - du moins provisoirement - le générateur d'ondes de choc.

5. Dispositif selon l'une des revendications précédentes, caractérisé en ce que les moyens (9, 10) pour la commande du générateur d'ondes de choc provoquent une réduction de l'énergie des ondes de choc produites par le générateur d'ondes de choc (1, 2, 3).

6. Dispositif selon l'une des revendications précédentes, caractérisé en ce que le générateur d'ondes de choc contient un émetteur d'impulsions (3) prédéterminant la séquence temporelle des ondes de choc avec une fréquence d'impulsions réglable.
